(19) **Europäisches Patentamt / European Patent Office / Office européen des brevets**

(11) **EP 4 702 845 A1**

(12) **EUROPEAN PATENT APPLICATION**
published in accordance with Art. 153(4) EPC

(43) Date of publication:
**04.03.2026 Bulletin 2026/10**

(21) Application number: **24797142.7**

(22) Date of filing: **25.04.2024**

(51) International Patent Classification (IPC):
**A23C 20/02** (2025.01)   **C12N 9/10** (2006.01)
**C12N 9/26** (2006.01)   **C12N 9/52** (2006.01)
**C12N 9/58** (2006.01)

(52) Cooperative Patent Classification (CPC):
**A23C 20/02; C12N 9/10; C12N 9/2408; C12N 9/52; C12N 9/58**

(86) International application number:
**PCT/JP2024/016326**

(87) International publication number:
**WO 2024/225397 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.04.2023   JP 2023073144**

(71) Applicants:
• **Amano Enzyme Inc.**
  **Nagoya-shi**
  **Aichi 460-8630 (JP)**
• **Amano Enzyme U.S.A. Co., Ltd.**
  **Elgin, Illinois 60124 (US)**

(72) Inventors:
• **HASHIMURA, Dai**
  **Elgin, Illinois 60124 (US)**
• **HENRY, Monica**
  **Elgin, Illinois 60124 (US)**

(74) Representative: **Müller-Boré & Partner**
**Patentanwälte PartG mbB**
**Friedenheimer Brücke 21**
**80639 München (DE)**

(54) **METHOD FOR PRODUCING PLANT-BASED CHEESE**

(57)   The purpose of the present invention is to provide a technique for producing a plant-based cheese having both a sufficient hardness in a non-heated state and heat-meltability. A plant-based cheese obtained by this method for producing a plant-based cheese has both sufficient hardness in a non-heated state and heat-meltability, the method including: (A) a step for subjecting a material containing a vegetable protein to hydrolysis and an enzymatic treatment with an enzyme selected from the group consisting of a glycosyltransferase, a maltotriohydrolase and a β-amylase; and (B) a step for adding starch thereto.

EP 4 702 845 A1

**Description**

Thnical Field

The present invention relates to a method for producing a plant-based cheese. More specifically, the present invention relates to a method for producing a plant-based cheese having both hardness in a non-heated state and melting property.

Background Art

For various reasons such as a recent health boom, countermeasures against allergy problems, and religious reasons, plant protein foods have become popular as alternatives to animal protein foods.

Since a plant protein material is greatly different in component composition from an animal protein material, various processing techniques have been studied in the creation of plant protein foods in order to bring flavor, texture, or the like closer to those of animal protein foods.

One of the characteristics of cheese using animal milk as a raw material is a characteristic of melting upon heating (this characteristic is hereinafter also referred to as "melting property"). This characteristic is related to milk fat and casein contained in animal milk and also contributes to the appetite for cheese. On the other hand, a plant-based cheese having a completely different protein composition does not inherently have melting property.

Therefore, a production method for imparting melting property to a plant-based cheese has been studied. For example, PTL 1 discloses that the melting property of a plant-based cheese to be obtained is improved by treating a material of a plant-based cheese with a protease.

Citation List

Patent Literature

PTL 1: WO 2022/181810 A

Summary of Invention

Technical Problem

A plant-based cheese obtained by the production method disclosed in PTL 1 exhibits excellent melting property, but the hardness decreases in exchange for the acquisition of melting property. Meanwhile, in consideration of the processing suitability of the plant-based cheese for shred processing, dice cutting processing, or the like, even a plant-based cheese having melting property is desired to have a favorable hardness in a non-heated state.

Therefore, an object of the present invention is to provide a technique for producing a plant-based cheese having both hardness in a non-heated state and melting property.

Solution to Problem

The present inventors have found that by subjecting a material containing a plant protein to a hydrolysis treatment and an enzymatic treatment with a specific carbohydrate-processing enzyme, both hardness in a non-heated state and melting property can be achieved. The present invention has been completed by conducting further studies based on these findings.

That is, the present invention provides inventions of the following aspects.

Item 1. A method for producing a plant-based cheese, the production method including: (A) a step for subjecting a material containing a plant protein to a hydrolysis treatment and an enzymatic treatment with an enzyme selected from the group consisting of a glycosyltransferase, a maltotriohydrolase, and a β-amylase; and (B) a step for mixing starch with the material.

Item 2. The production method according to item 1, in which the glycosyltransferase is a cyclodextrin-producing enzyme.

Item 3. The production method according to item 1 or 2, in which the hydrolysis treatment is an enzymatic treatment with a protease.

Item 4. The production method according to item 3, in which the protease is a filamentous fungus-derived protease and/or a bacteria-derived protease.

Item 5. The production method according to any one of items 1 to 4, including a step for deactivating the enzyme used in the step (A) after the step (A) and before the step (B).

Item 6. The production method according to any one of items 3 to 5, in which a used amount of the protease per 1 g of the plant protein in the step (A) is 5 to 300000 U.

Item 7. The production method according to any one of items 1 to 6, in which a used amount of the glycosyltransferase per 1 g of the plant protein in the step (A) is 0.5 to 15000 U.

Item 8. The production method according to any one of items 1 to 7, in which a used amount of the maltotriohydrolase per 1 g of the plant protein in the step (A) is 0.01 to 30 U.

Item 9. The production method according to any one of items 1 to 8, in which a used amount of the β-amylase per 1 g of the plant protein in the step (A) is 0.005 to 15 U.

Item 10. The production method according to any one of items 1 to 9, in which a used amount of the starch in the step (B) is 0.2 to 1 part by weight per 1 part by weight of the plant protein.

Item 11. A method for imparting hardness and melting property of a plant-based cheese, the imparting method including, in production of a plant-based cheese: (A) a step for subjecting a material containing a plant protein to a hydrolysis treatment and an enzymatic treatment with an enzyme selected from the group consisting of a glycosyltransferase, a maltotriohydrolase, and a β-amylase; and (B) a step for mixing starch with the material.

Item 12. A plant-based cheese obtained by the production method according to any one of items 1 to 10.

Item 13. A method for producing a plant-based cheese, the production method including: a step for subjecting a hydrolyzed material containing a plant protein to an enzymatic treatment with an enzyme selected from the group consisting of a glycosyltransferase, a maltotriohydrolase, and a β-amylase; and a step for mixing starch with the material.

Advantageous Effects of Invention

According to the present invention, there is provided a technique for producing a plant-based cheese having both hardness in a non-heated state and melting property.

Description of Embodiments

1. Method for Producing Plant-Based Cheese

A method for producing a plant-based cheese of the present invention includes (A) a step for subjecting a material containing a plant protein to a hydrolysis treatment and an enzymatic treatment with an enzyme selected from the group consisting of a glycosyltransferase, a maltotriohydrolase, and a β-amylase (hereinafter, also referred to as "step (A)"); and (B) a step for mixing starch with the material (hereinafter, also referred to as "step (B)"). This feature makes it possible to achieve both hardness in a non-heated state and melting property in a plant-based cheese to be obtained. In a preferred embodiment, more excellent cohesiveness and/or a raw material flavor reducing effect can be imparted to a plant-based cheese to be obtained. Hereinafter, the method for producing a plant-based cheese of the present invention will be specifically described.

1-1. Step (A)

In the step (A), a material containing a plant protein is subjected to a hydrolysis treatment and an enzymatic treatment with an enzyme selected from the group consisting of a glycosyltransferase, a maltotriohydrolase, and a β-amylase (the treatment with an enzyme using these carbohydrates as a substrate is hereinafter also referred to as "predetermined enzymatic treatment"). More specifically, in the step (A), a material containing a plant protein is subjected to a hydrolysis treatment and a predetermined enzymatic treatment to obtain an enzyme-treated product. The hydrolysis treatment and the predetermined enzymatic treatment may be performed simultaneously or sequentially. When these treatments are sequentially performed, any treatment may be performed first. From the viewpoint of obtaining excellent compatibility between hardness and melting property, and/or, in addition to this viewpoint, from the viewpoint of also obtaining excellent cohesiveness and/or a raw material flavor reducing effect, it is preferable that these treatments are performed by performing the hydrolysis treatment first or simultaneously, and it is more preferable that the hydrolysis is performed first.

1-1-1. Material Containing Plant Protein

The material containing a plant protein is a mixture to be subjected to a hydrolysis treatment and an enzymatic treatment, and contains a plant protein and starch.

The origin plants of the plant protein are not particularly limited, and examples thereof include legumes such as peas, soybean, fava bean, chickpea, and lentil; cereals such as barley, wheat, oat, rice, buckwheat, Japanese barnyard millet, and foxtail millet; and nuts such as almond, cashew nut, hazelnut, pecan nut, macadamia nut, pistachio, walnut, brazil nut, peanut, and coconut and the like. The plant proteins derived from these plants may be used singly, or two or more types having different origins may be used in combination.

Among them, from the viewpoint of obtaining excellent compatibility between hardness and melting property, and/or, in addition to this viewpoint, from the viewpoint of also obtaining excellent cohesiveness and/or a raw material flavor reducing effect, a bean protein is preferable, a protein from peas, fava bean, chickpea, or lentil is more preferable, and a protein of peas is still more preferable.

The content of the plant protein in the material containing a plant protein is not particularly limited, and is, for example, 0.1 wt% or more, 0.5 wt% or more, or 1 wt% or more, and is preferably 3 wt% or more, more preferably 5 wt% or more, still more preferably 10 wt% or more, further preferably 15 wt% or more, even more preferably 18 wt% or more, and particularly preferably 20 wt% or more, from the viewpoint of obtaining excellent compatibility between hardness and melting property, and/or, in addition to this viewpoint, from the viewpoint of also obtaining excellent cohesiveness and/or a raw material flavor reducing effect. The upper limit of the content of the plant protein in the material containing a plant protein is also not particularly limited, and is, for example, 50 wt% or less or 40 wt% or less, and is preferably 30 wt% or less, more preferably 25 wt% or less, and still more preferably 23 wt% or less, from the viewpoint of obtaining excellent compatibility between hardness and melting property, and/or, in addition to this viewpoint, from the viewpoint of also obtaining excellent cohesiveness and/or a raw material flavor reducing effect.

The origin plants of the starch may be the same as or different from the origin plants of the plant protein. From the viewpoint of obtaining excellent compatibility between hardness and melting property, and/or, in addition to this viewpoint, from the viewpoint of also obtaining excellent cohesiveness and/or a raw material flavor reducing effect, the origin plants of the starch are preferably the same as the origin plants of the plant protein.

The content of the starch in the material containing a plant protein is not particularly limited, and is, for example, 0.01 wt% or more, and is preferably 0.05 wt% or more or 0.1 wt% or more, more preferably 0.3 wt% or more, still more preferably 0.5 wt% or more, further preferably 0.7 wt% or more, and particularly preferably 0.9 wt% or more, from the viewpoint of obtaining excellent compatibility between hardness and melting property, and/or, in addition to this viewpoint, from the viewpoint of also obtaining excellent cohesiveness and/or a raw material flavor reducing effect. The upper limit of the content of the starch in the material containing a plant protein is also not particularly limited, and is, for example, 10.0 wt% or less. Since the production method of the present invention is excellent in the effect of improving hardness and stretchability, hardness can be effectively improved even when the content of the starch is relatively small. From such a viewpoint, the upper limit of the range of the content of starch in the material to be enzymatically reacted is preferably 5.0 wt% or less, more preferably 3.0 wt% or less, still more preferably 2.0 wt% or less, further preferably 1.5 wt% or less, and particularly preferably 1.3 wt% or less.

The ratio of the plant protein and the starch (the content of the starch/the content of the plant protein) in the material containing a plant protein is determined by the content of each component, and is preferably 0.01 or more, more preferably 0.02 or more, still more preferably 0.03 or more, and further preferably 0.04 or more, from the viewpoint of obtaining excellent compatibility between hardness and melting property, and/or, in addition to this viewpoint, from the viewpoint of also obtaining excellent cohesiveness and/or a raw material flavor reducing effect. The upper limit of the ratio is also not particularly limited, and is preferably 0.09 or less, still more preferably 0.07 or less, and further preferably 0.06 or less, from the viewpoint of obtaining excellent compatibility between hardness and melting property, and/or, in addition to this viewpoint, from the viewpoint of also obtaining excellent cohesiveness and/or a raw material flavor reducing effect.

The material containing a plant protein can contain, as components other than the plant protein and the starch, any material components (hereinafter, also referred to as "other material components") used for a plant-based cheese. Examples of the other material components include water, an organic acid, plant fat and oil, thickening polysaccharides, common salt, a calcium salt and the like, and one or more of these components can be contained in the material containing a plant protein. Note that the material containing a plant protein typically contains water as other material components.

From the viewpoint of obtaining excellent compatibility between hardness and melting property, and/or, in addition to this viewpoint, from the viewpoint of also obtaining excellent cohesiveness and/or a raw material flavor reducing effect, the material containing a plant protein preferably contains an organic acid and water among the other material components. Note that, when the present step (A) and the step (B) described later are performed in this order, the organic acid may be mixed with starch into the enzyme-treated product in the step (B), instead of being contained in the material containing a plant protein in the present step (A). From the viewpoint of obtaining excellent compatibility between hardness and melting property, and/or, in addition to this viewpoint, from the viewpoint of also obtaining excellent cohesiveness and/or a raw material flavor reducing effect, when the present step (A) and the step (B) described later are performed in this order, it is more preferable that the organic acid is contained in the material containing a plant protein in the present step (A).

The organic acid is not particularly limited, and examples thereof include lactic acid, citric acid, acetic acid, succinic acid, and the like. These organic acids may be used singly or in combination of a plurality of kinds thereof. Among these organic acids, lactic acid is preferable.

When the material containing a plant protein contains an organic acid, the content of the organic acid in the material is not particularly limited, and from the viewpoint of obtaining excellent compatibility between hardness and melting property, and/or, in addition to this viewpoint, from the viewpoint of also obtaining excellent cohesiveness and/or a raw material flavor reducing effect, the content thereof is, for example, 0.1 to 1 wt% and more preferably 0.3 to 0.5 wt%.

When the material containing a plant protein contains water, the content of the water in the material is not particularly limited, and from the viewpoint of obtaining excellent compatibility between hardness and melting property, and/or, in addition to this viewpoint, from the viewpoint of also obtaining excellent cohesiveness and/or a raw material flavor reducing effect, the content thereof is, for example, 50 to 90 wt% or 60 to 85 wt%, preferably 65 to 80 wt%, and more preferably 70 to 75 wt%.

In the present step, among the other material components described above, components (plant fat and oil, thickening polysaccharides, common salt, and calcium salt) other than the organic acid and water are preferably not contained in the material containing a plant protein when the present step (A) and the step (B) described later are performed in this order. However, the present invention does not exclude a case where components other than the organic acid and water are contained in the material containing a plant protein. For example, when the present step (A) and the step (B) described later are performed simultaneously, components other than the organic acid and water can be contained in the material containing a plant protein. Note that more specific examples of the plant fat and oil, thickening polysaccharides (other than starch), starch, common salt, and calcium salt will be described in detail in "1-2. Step (B)".

Preferable specific forms of the material containing a plant protein include a mixture prepared by mixing a plant raw material (an organ of an origin plant of a plant protein or a part thereof), a dry powder thereof, or a processed raw material, which is obtained by such as removing at least a part of components other than the plant protein from the plant raw material or the dry powder thereof to increase the content of the plant protein, with other material components (preferably, water and an organic acid).

1-1-2. Hydrolysis Treatment

A means for the hydrolysis treatment is not particularly limited as long as it is a means capable of hydrolyzing a protein, and may be either an enzymatic treatment or a chemical treatment. In the present invention, as the means for the hydrolysis treatment, an enzymatic treatment is preferably used. As the enzyme used for the enzymatic treatment, an enzyme having a protein hydrolyzing action can be used without particular limitation. In the present invention, from the viewpoint of obtaining excellent compatibility between hardness and melting property, and/or, in addition to this viewpoint, from the viewpoint of also obtaining excellent cohesiveness and/or a raw material flavor reducing effect, a protease is preferably used.

Protease

In the present invention, the protease refers to an endo-type peptidase. As the protease, a protease derived from any organism can be used, and examples thereof include proteases derived from filamentous fungi such as the genera Aspergillus, Mucor, Neurospora, Penicillium, Rhizomucor, Rhizopus, and Sclerotinia; proteases derived from yeast of the genus Saccharomyces; proteases derived from bacteria such as the genera Bacillus and Geobacillus; and proteases derived from actinomycetes such as the genus Streptomyces. These proteases may be used singly or in combination of a plurality of kinds thereof.

Among these proteases, from the viewpoint of obtaining excellent compatibility between hardness and melting property, and/or, in addition to this viewpoint, from the viewpoint of also obtaining excellent cohesiveness and/or a raw material flavor reducing effect, a filamentous fungus-derived protease and/or a bacteria-derived protease are preferably used, and a filamentous fungus-derived protease and a bacteria-derived protease are more preferably used in combination.

From the viewpoint of obtaining excellent compatibility between hardness and melting property, and/or, in addition to this viewpoint, from the viewpoint of also obtaining excellent cohesiveness and/or a raw material flavor reducing effect, examples of the filamentous fungus-derived protease include preferably a protease derived from the genus Aspergillus, more preferably a protease derived from Aspergillus oryzae or Aspergillus niger, and still more preferably a protease derived from Aspergillus oryzae. From the viewpoint of obtaining excellent compatibility between hardness and melting property, and/or, in addition to this viewpoint, from the viewpoint of also obtaining excellent cohesiveness and/or a raw material flavor reducing effect, examples of the bacteria-derived protease include preferably proteases derived from Bacillus stearothermophilus, Bacillus licheniformis, Bacillus amyloliquefaciens, and the genus Geobacillus thereof, and more preferably a protease derived from Geobacillus stearothermophilus.

The used amount of the protease is not particularly limited, and the protease can be used in an amount per 1 g of the plant protein of, for example, 5 to 300000 U, 50 to 25000 U, 100 to 20000 U, or 240 to 10000 U. From the viewpoint of obtaining excellent compatibility between hardness and melting property, and/or, in addition to this viewpoint, from the viewpoint of also obtaining excellent cohesiveness and/or a raw material flavor reducing effect, the protease can be used in an amount per 1 g of plant protein of preferably 400 to 8000 U, more preferably 700 to 6000 U, and still more preferably 1100 to 3600 U, 1100 to 2900 U, 1100 to 2300 U, 1150 to 1800 U, or 1200 U to 1500 U.

When a filamentous fungus-derived protease is used as the protease, the used amount of the filamentous fungus-derived protease is not particularly limited, and the filamentous fungus-derived protease can be used so that the protease activity (pH 3.0) per 1 g of the plant protein is, for example, 10 to 200000 U, 100 to 100000 U, or 190 to 20000 U, and from the viewpoint of obtaining excellent compatibility between hardness and melting property, and/or, in addition to this viewpoint, from the viewpoint of also obtaining excellent cohesiveness and/or a raw material flavor reducing effect, the filamentous fungus-derived protease can be used so that the protease activity is preferably 300 to 10000 U or 400 to 7500 U, more preferably 500 to 5000 U, and still more preferably 900 to 3000 U, 900 to 2500 U, 900 to 2000 U, 900 to 1500 U, or 900 to 1000 U.

When a bacteria-derived protease is used as the protease, the used amount of the bacteria-derived protease is not particularly limited, and the bacteria-derived protease can be used so that the protease activity (pH 8.0) per 1 g of the plant protein is, for example, 5 to 60000 U, 30 to 10000 U, or 50 to 6000 U, and from the viewpoint of obtaining excellent compatibility between hardness and melting property, and/or, in addition to this viewpoint, from the viewpoint of also obtaining excellent cohesiveness and/or a raw material flavor reducing effect, the bacteria-derived protease can be used so that the protease activity is preferably 100 to 3000 U, more preferably 200 to 1000 U, and still more preferably 250 to 600 U or 250 to 300 U.

When a filamentous fungus-derived protease and a bacteria-derived protease are used in combination as the protease, the ratio of the used amounts of the filamentous fungus-derived protease and the bacteria-derived protease is determined according to the used amount of each protease described above, and from the viewpoint of obtaining excellent compatibility between hardness and melting property, and/or, in addition to this viewpoint, from the viewpoint of also obtaining excellent cohesiveness and/or a raw material flavor reducing effect, the used amount of the bacteria-derived protease with respect to 1 U of the filamentous fungus-derived protease is preferably 0.05 to 5 U or 0.1 to 3 U, more preferably 0.15 to 2 U, still more preferably 0.2 to 1 U, and further preferably 0.25 to 0.4 U.

Note that the protease activity is measured using casein as a substrate by the Folin method. That is, the protease activity is an enzyme activity in which an enzymatic reaction is performed using casein as a substrate by a conventional method, and the amount of enzyme that causes an increase in colored materials by Folin's reagent corresponding to 1 $\mu$g of tyrosine per minute is defined as 1 unit (1 U).

Treatment Conditions, etc.

Conditions for the hydrolysis treatment can be appropriately set by those skilled in the art depending on the hydrolysis treatment means. Even when the hydrolysis treatment is performed using a protease, those skilled in the art can appropriately set conditions under which the protease effectively acts on a material containing a plant protein.

The temperature of the enzymatic treatment with a protease is not particularly limited, and can be appropriately determined by those skilled in the art according to the optimal temperature of the enzyme to be used, and the like (or when the treatment is performed simultaneously with the predetermined enzymatic treatment, the optimal temperature of the enzyme used in the predetermined enzymatic treatment and the like are also considered), and is, for example, 35 to 65°C, preferably 40 to 60°C, and more preferably 45 to 55°C.

The pH (at 25°C) of the enzymatic treatment with a protease is not particularly limited, and can be appropriately determined by those skilled in the art according to the optimal pH of the enzyme to be used, and the like (or when the treatment is performed simultaneously with the predetermined enzymatic treatment, the optimal pH of the enzyme used in the predetermined enzymatic treatment and the like are also considered), and is, for example, 3.8 to 7.2, preferably 4 to 7, more preferably 4 to 6, and still more preferably 4.2 to 5.

The enzymatic treatment time with a protease is also not particularly limited, and may be appropriately determined according to the preparation scale of the material containing a plant protein, and the like, and is, for example, 30 seconds to 24 hours, preferably 1 minute to 12 hours, and more preferably 3 minutes to 6 hours, 5 minutes to 3 hours, 10 minutes to 2 hours, or 15 minutes to 1 hour.

1-1-3. Enzymatic Treatment

The enzymatic treatment is performed with an enzyme selected from the group consisting of a glycosyltransferase, a maltotriohydrolase, and a $\beta$-amylase. These enzymes may be used singly or in combination of a plurality of kinds thereof. Among these enzymes, from the viewpoint of obtaining excellent compatibility between hardness and melting property, and/or, in addition to this viewpoint, from the viewpoint of also obtaining excellent cohesiveness and/or a raw material flavor reducing effect, a glycosyltransferase and/or a maltotriohydrolase are preferable, and a glycosyltransferase is more preferable.

Glycosyltransferase

In the present invention, the glycosyltransferase means hexosyltransferase (E.C.2.4.1). Examples of the glycosyltransferase (hexosyltransferase) include a cyclodextrin-producing enzyme (E.C.2.4.1.19), a 1,4-α-glucan branching enzyme (E.C.2.4.1.18), a 4-α-glucanotransferase (E.C.2.4.1.25), and the like. These glycosyltransferases may be used singly or in combination of a plurality of kinds thereof. Among these glycosyltransferases, from the viewpoint of obtaining excellent compatibility between hardness and melting property, and/or, in addition to this viewpoint, from the viewpoint of also obtaining excellent cohesiveness and/or a raw material flavor reducing effect, a cyclodextrin-producing enzyme is preferable.

As the cyclodextrin-producing enzyme, a cyclodextrin-producing enzyme derived from any organism can be used, and examples thereof include cyclodextrin-producing enzymes derived from the genera Anoxybacillus, Bacillus, Geobacillus, Paenibacillus, Klebsiella, Thermoanaerobactor, and Brevibacterium, and more specific examples thereof include cyclodextrin-producing enzymes derived from Anoxybacillus caldiproteolyticus (also including those previously classified as Geobacillus stearothermophilus), Bacillus stearothermophilus, Bacillus megaterium, Bacillus circulans, Bacillus macerans, Bacillus ohbensis, Bacillus clarkii, Geobacillus stearothermophilus, Paenibacillus macerans, and Klebsiella pneumoniae. These cyclodextrin-producing enzymes may be used singly or in combination of a plurality of kinds thereof.

Among these cyclodextrin-producing enzymes, from the viewpoint of obtaining excellent compatibility between hardness and melting property, and/or, in addition to this viewpoint, from the viewpoint of also obtaining excellent cohesiveness and/or a raw material flavor reducing effect, cyclodextrin-producing enzymes derived from the genera Anoxybacillus, Bacillus, Paenibacillus, and Geobacillus are preferable, cyclodextrin-producing enzymes derived from Anoxybacillus caldiproteolyticus, Paenibacillus macerans, and Geobacillus stearothermophilus are more preferable, and cyclodextrin-producing enzymes derived from Anoxybacillus caldiproteolyticus are still more preferable.

The used amount of the glycosyltransferase is not particularly limited, and the glycosyltransferase can be used in an amount per 1 g of the starch of, for example, 0.5 to 15000 U or 5 to 10000 U. From the viewpoint of obtaining excellent compatibility between hardness and melting property, and/or, in addition to this viewpoint, from the viewpoint of also obtaining excellent cohesiveness and/or a raw material flavor reducing effect, the cyclodextrin-producing enzyme can be used in an amount per 1 g of the starch of preferably 10 to 1000 U or 20 to 300 U, more preferably 30 to 150 U, still more preferably 35 to 90 U, 35 to 85 U, 35 to 80 U, 35 to 75 U, 35 to 70 U, 36 to 65 U, 37 to 60 U, 38 to 55 U, or 39 to 50 U, and from the viewpoint of obtaining a more excellent raw material flavor reducing effect, the cyclodextrin-producing enzyme can be used in an amount per 1 g of the starch of more preferably 62 to 150 U, and still more preferably 65 to 100 U, 70 to 95 U, 75 to 90 U, or 78 to 85 U.

Note that cyclodextrin-producing enzyme activity is an enzyme activity in which an enzymatic reaction is performed using starch as a substrate by a conventional method, and the amount of enzyme that causes a decrease in blue iodine color of starch by 1% per minute is defined as 1 unit (U).

Maltotriohydrolase

As the maltotriohydrolase (maltotriose-producing amylase) (EC3.2.1.11), a maltotriohydrolase derived from any organism can be used, and examples thereof include maltotriohydrolases derived from the genera Microbacterium and Cellulosimicrobium. Examples of the maltotriohydrolase of the genus Microbacterium include Microbacterium imperiale. These maltotriohydrolases may be used singly or in combination of a plurality of kinds thereof.

Among these maltotriohydrolases, from the viewpoint of obtaining excellent compatibility between hardness and melting property, and/or, in addition to this viewpoint, from the viewpoint of also obtaining excellent cohesiveness and/or a raw material flavor reducing effect, maltotriohydrolase derived from the genus Microbacterium is preferably used, and maltotriohydrolase derived from Microbacterium imperiale is preferably used.

The used amount of the maltotriohydrolase is not particularly limited, and the maltotriohydrolase can be used in an amount per 1 g of the starch of, for example, 0.01 to 30 U or 0.05 to 10 U. From the viewpoint of obtaining excellent compatibility between hardness and melting property, and/or, in addition to this viewpoint, from the viewpoint of also obtaining excellent cohesiveness and/or a raw material flavor reducing effect, the maltotriohydrolase can be used in an amount per 1 g of the starch of preferably 0.1 to 5 U, more preferably 0.3 to 3 U, still more preferably 0.5 to 2 U, further preferably 0.8 to 1.8 U, and particularly preferably 1 to 1.5 U.

Note that the maltotriohydrolase activity is an enzyme activity in which an enzymatic reaction is performed using starch as a substrate by a conventional method, and the amount of enzyme that produces a reducing sugar corresponding to 1 μmol of glucose per minute is defined as 1 unit.

β-Amylase

As the β-amylase, a β-amylase derived from any organism can be used, and examples thereof include β-amylase derived from plants such as wheat, barley, and soybean, and β-amylase derived from microorganisms of the genera Bacillus, Streptomyces, Pseudomonas, and the like. Examples of the β-amylase derived from the genus Bacillus include Bacillus flexus, Bacillus megaterium, Bacillus polymyxa, Bacillus circulans, and the like. These β-amylases may be used singly or in combination of a plurality of kinds thereof.

Among these β-amylases, from the viewpoint of obtaining excellent compatibility between hardness and melting property, and/or, in addition to this viewpoint, from the viewpoint of also obtaining excellent cohesiveness and/or a raw material flavor reducing effect, β-amylase derived from Bacillus is preferable, β-amylases derived from Bacillus flexus, Bacillus megaterium, Bacillus polymyxa, and Bacillus circulans are more preferable, and β-amylase derived from Bacillus flexus is still more preferable.

The used amount of the β-amylase is not particularly limited, and the β-amylase can be used in an amount per 1 g of the starch of, for example, 0.005 to 15 U or 0.025 to 5 U. From the viewpoint of obtaining excellent compatibility between hardness and melting property, and/or, in addition to this viewpoint, from the viewpoint of also obtaining excellent cohesiveness and/or a raw material flavor reducing effect, the β-amylase can be used in an amount per 1 g of the starch of preferably 0.05 to 2.5 U, more preferably 0.15 to 1.5 U, still more preferably 0.25 to 1 U, further preferably 0.4 to 0.9 U, and particularly preferably 0.5 to 0.75 U.

Note that the activity of the β-amylase is an enzyme activity in which an enzymatic reaction is performed using potato starch as a substrate, and the amount of enzyme that causes an increase in reducing power corresponding to 1 mg of glucose per minute is defined as 1 unit (1 U).

Treatment Conditions, etc.

The temperature of the enzymatic treatment with an enzyme using the carbohydrate as a substrate is not particularly limited, and can be appropriately determined by those skilled in the art according to the optimal temperature of the enzyme to be used, and the like (or when the treatment is performed simultaneously with the hydrolysis treatment using a protease, the optimal temperature of the protease and the like are also considered), and is, for example, 35 to 65°C, preferably 40 to 60°C, and more preferably 45 to 55°C.

The pH (at 25°C) of the enzymatic treatment with an enzyme using the carbohydrate as a substrate is not particularly limited, and can be appropriately determined by those skilled in the art according to the optimal pH of the enzyme to be used, and the like (or when the treatment is performed simultaneously with the hydrolysis treatment using a protease, the optimal pH of the protease and the like are also considered), and is, for example, 3.8 to 7.2, preferably 4 to 7, more preferably 4 to 6, and still more preferably 4.2 to 5.

The enzymatic treatment time with an enzyme using the carbohydrate as a substrate is also not particularly limited, and may be appropriately determined according to the preparation scale of the material containing a plant protein, and the like, and is, for example, 30 seconds to 24 hours, preferably 1 minute to 12 hours, and more preferably 3 minutes to 6 hours, 5 minutes to 3 hours, 10 minutes to 2 hours, or 15 minutes to 1 hour.

The method for producing a plant-based cheese of the present invention may include a step for subjecting a hydrolyzed material containing a plant protein to an enzymatic treatment with an enzyme selected from the group consisting of a glycosyltransferase, a maltotriohydrolase, and a β-amylase; and a step for mixing starch with the material. In this case, the hydrolyzed material containing a plant protein is prepared as a material obtained by already subjecting the material described in the above "1-1-1. Material Containing Plant Protein" to the treatment described in the above "1-1-2. Hydrolysis Treatment". The enzyme-treated product can be obtained by subjecting the hydrolyzed material containing a plant protein to the predetermined enzymatic treatment described in the above "1-1-3. Enzymatic Treatment".

1-2. Step (B)

In the step (B), starch is mixed. The step (B) may be performed after the step (A), may be performed simultaneously with the step (A), or may be performed before the step (A). In the step (B) performed after the step (A), starch is mixed with the enzyme-treated product obtained in the step (A). When the enzyme deactivation step described later is performed, starch is mixed with the deactivated product of the enzyme-treated product. In the step (B) performed simultaneously with the step (A), starch is mixed with a material containing a plant protein to be treated in the step (A). In the step (B) performed before the step (A), starch is mixed at the time of preparing a material containing a plant protein. From the viewpoint of obtaining excellent compatibility between hardness and melting property, and/or, in addition to this viewpoint, from the viewpoint of also obtaining excellent cohesiveness and/or a raw material flavor reducing effect, the step (B) is preferably performed after the step (A) or simultaneously with the step (A); when the hydrolysis treatment and the predetermined enzymatic treatment are performed in this order in the step (A), the step (B) is preferably performed after the step (A); and when the hydrolysis treatment and the predetermined enzymatic treatment are simultaneously performed in the step (A), the step (B) is preferably performed simultaneously with the step (A). The step (A) and the step (B) are completed to obtain a plant-based cheese dough.

The origin plants of the starch are not particularly limited, and examples thereof include cassava, potato, sweet potato, arrowroot, and the like. The starches derived from these plants may be used singly, or two or more types having different origins may be used in combination.

Among these, from the viewpoint of obtaining excellent compatibility between hardness and melting property, and/or, in addition to this viewpoint, from the viewpoint of also obtaining excellent cohesiveness and/or a raw material flavor reducing effect, cassava starch (tapioca starch) and potato starch are preferable, and cassava starch (tapioca starch) is more preferable.

The mixing amount of the starch is not particularly limited as long as the effect of the present invention can be obtained, and the mixing amount is, for example, 4 wt% or more in terms of the content in the plant-based cheese dough after mixing, and from the viewpoint of obtaining excellent compatibility between hardness and melting property, and/or, in addition to this viewpoint, from the viewpoint of also obtaining excellent cohesiveness and/or a raw material flavor reducing effect, the mixing amount is preferably 5 wt% or more, more preferably 6 wt% or more, and still more preferably 7 wt% or more.

The upper limit of the mixing amount of the starch is also not particularly limited, and is, for example, 20 wt% or less in terms of the content of the plant-based cheese dough after mixing. Since the production method of the present invention is excellent in compatibility between hardness and melting property, it is possible to effectively achieve both hardness and melting property even when the content of the starch is relatively small. From such a viewpoint, the mixing amount of the starch is preferably 17 wt% or less, more preferably 15 wt% or less, still more preferably 13 wt% or less, and further preferably 12 wt% or less.

The ratio of the plant protein used in the preparation of the enzyme-treated product in the step (A) and the starch in the present step is determined by the content of each of the above components, and the content of the starch per 1 part by weight of the plant protein used in the preparation of the enzyme-treated product is preferably 0.2 to 1 part by weight, more preferably 0.4 to 0.9 parts by weight, and still more preferably 0.6 to 0.7 parts by weight.

In the step (B), in addition to the starch described above, any other material components used for the production of a plant-based cheese can be blended. Examples of such other material components include plant fat and oil, thickening polysaccharides, common salt, a calcium salt, an organic acid, and the like.

The plant fat and oil are not particularly limited, and examples thereof include canola oil (rapeseed oil), coconut oil, corn oil, olive oil, soybean oil, peanut oil, walnut oil, almond oil, sesame oil, cottonseed oil, sunflower seed oil, safflower oil, flaxseed oil, palm oil, palm kernel oil, palm fruit oil, babassu oil, shea butter, mango butter, cocoa butter, wheat germ oil, rice bran oil, and the like. These plant fats and oils may be used singly or in combination of two or more kinds thereof. Among these plant fats and oils, from the viewpoint of obtaining excellent compatibility between hardness and melting property, and/or, in addition to this viewpoint, from the viewpoint of also obtaining excellent cohesiveness and/or a raw material flavor reducing effect, canola oil (rapeseed oil), coconut oil, and sunflower oil are preferable, and canola oil (rapeseed oil) and coconut oil are more preferable.

In the case of mixing plant fat and oil, the mixing amount thereof is not particularly limited, and from the viewpoint of obtaining excellent compatibility between hardness and melting property, and/or, in addition to this viewpoint, from the viewpoint of also obtaining excellent cohesiveness and/or a raw material flavor reducing effect, the mixing amount is, for example, 4 to 30 wt%, preferably 8 to 25 wt%, and more preferably 12 to 20 wt% in terms of the content of the plant-based cheese dough after mixing.

The thickening polysaccharides are not particularly limited, and examples thereof include locust bean gum, guar gum, carrageenan, xanthan gum, tragacanth gum, tamarind seed gum, pectin, gum arabic, curdlan, tara gum, gellan gum, ghatti gum, CMC (carboxymethyl cellulose), sodium alginate, pullulan, and the like, and carrageenan and the like are preferable. These thickening polysaccharides may be used singly or in combination with two or more kinds thereof. Among these thickening polysaccharides, from the viewpoint of obtaining excellent compatibility between hardness and melting property, and/or, in addition to this viewpoint, from the viewpoint of also obtaining excellent cohesiveness and/or a raw material flavor reducing effect, carrageenan is preferable.

In the case of mixing thickening polysaccharides, the mixing amount thereof is not particularly limited, and from the viewpoint of obtaining excellent compatibility between hardness and melting property, and/or, in addition to this viewpoint, from the viewpoint of also obtaining excellent cohesiveness and/or a raw material flavor reducing effect, the mixing amount is, for example, 0.1 to 2 wt%, more preferably 0.4 to 1.5 wt%, and still more preferably 0.6 to 1 wt% in terms of the content in the plant-based cheese dough after mixing.

In the case of mixing common salt, the mixing amount thereof is not particularly limited, and from the viewpoint of obtaining excellent compatibility between hardness and melting property, and/or, in addition to this viewpoint, from the viewpoint of also obtaining excellent cohesiveness and/or a raw material flavor reducing effect, the mixing amount is, for example, 0.1 to 2 wt%, and more preferably 0.5 to 0.7 wt% in terms of the content of the plant-based cheese dough after mixing.

The calcium salt is not particularly limited, and examples thereof include calcium gluconate, calcium lactate, calcium phosphate, and the like. These calcium salts may be used singly or in combination with two or more kinds thereof. Among these calcium salts, from the viewpoint of obtaining excellent compatibility between hardness and melting property, and/or, in addition to this viewpoint, from the viewpoint of also obtaining excellent cohesiveness and/or a raw material flavor reducing effect, calcium phosphate is preferable.

In the case of mixing a calcium salt, the mixing amount thereof is not particularly limited, and from the viewpoint of obtaining excellent compatibility between hardness and melting property, and/or, in addition to this viewpoint, from the viewpoint of also obtaining excellent cohesiveness and/or a raw material flavor reducing effect, the mixing amount is, for example, 0.5 to 4 wt%, more preferably 0.9 to 2.5 wt%, and more preferably 1.2 to 1.8 wt% in terms of the content of the plant-based cheese dough after mixing.

The organic acid is not particularly limited, and specific examples thereof include lactic acid, citric acid, acetic acid, succinic acid, and the like as described in the above "1-1-1. Material Containing Plant Protein". These organic acids may be used singly or in combination of a plurality of kinds thereof. Among these organic acids, lactic acid is preferable.

In the case of mixing an organic acid, the mixing amount thereof is not particularly limited, and from the viewpoint of obtaining excellent compatibility between hardness and melting property, and/or, in addition to this viewpoint, from the viewpoint of also obtaining excellent cohesiveness and/or a raw material flavor reducing effect, the mixing amount is, for example, 0.1 to 1 wt%, and more preferably 0.3 to 0.5 wt% in terms of the content of the plant-based cheese dough after mixing.

### 1-3. Enzyme Deactivation Step

In the present invention, when the step (A) and the step (B) are performed in this order, it is preferable to include a step for deactivating the enzyme used in the step (A) (hereinafter, also referred to as "enzyme deactivation step") after the step (A) and before the step (B).

In the enzyme deactivation method, the enzyme-treated product obtained in the step (A) may be subjected to enzyme deactivation conditions. As enzyme deactivation conditions, conditions for denaturing the enzyme used in the step (A) (specifically, an enzyme using a carbohydrate as a substrate, or an enzyme using a carbohydrate as a substrate and a protease) may be appropriately selected, and heat deactivation is typically performed. Specific temperature conditions for the heat deactivation may be set according to the thermal characteristics of the enzyme actually used in the step (A), and examples thereof include 70°C or higher, preferably 80°C or higher, and more preferably 85°C or higher. The heat deactivation time is not particularly limited, and is, for example, 10 to 20 minutes.

### 1-4. Other Steps

The production method of the present invention can further include any other step for obtaining a plant-based cheese in addition to the step (A) and the step (B), and the enzyme deactivation step performed as necessary.

Examples of the other steps include a step for heating the plant-based cheese dough obtained in the step (B) to prepare the plant-based cheese, and a step for cooling the obtained plant-based cheese.

The heating temperature in the step for heating the plant-based cheese dough is, for example, 70 to 110°C, preferably 80 to 100°C, and more preferably 85 to 95°C.

The heating time in the step for heating the plant-based cheese dough is not particularly limited, and is, for example, 5 to 30 minutes, and preferably 10 to 20 minutes.

In the cooling step, the obtained plant-based cheese can be appropriately cooled in a state of being filled in a container having an appropriate size. The temperature in the cooling step is not particularly limited, and is, for example, 2 to 5°C.

### 2. Method for Imparting Hardness and Melting Property of Plant-Based Cheese

As described above, by performing the step (A) and the step (B) in the production of a plant-based cheese, both hardness in a non-heated state and melting property can be imparted to a plant-based cheese to be obtained. Therefore, the present invention also provides a method for imparting hardness and melting property of a plant-based cheese, the imparting method including, in production of a plant-based cheese: (A) a step for subjecting a material containing a plant protein to a hydrolysis treatment and an enzymatic treatment with an enzyme selected from the group consisting of a glycosyltransferase, a maltotriohydrolase, and a β-amylase; and (B) a step for mixing starch with the material.

In the method for imparting melting property and hardness of the present invention, the type, used amount, and the like of the component to be used are as described in the section of "1. Method for Producing Plant-Based Cheese".

3. Plant-Based Cheese

A plant-based cheese to be obtained by the production method described in the section of "1. Method for Producing Plant-Based Cheese" has both hardness in a non-heated state and melting property. Therefore, the present invention also provides a plant-based cheese obtained by a method for producing a plant-based cheese, the production method including: (A) a step for subjecting a material containing a plant protein to a hydrolysis treatment and an enzymatic treatment with an enzyme selected from the group consisting of a glycosyltransferase, a maltotriohydrolase, and a β-amylase; and (B) a step for mixing starch with the material.

The hardness of the plant-based cheese of the present invention is, for example, 3.2 N or more, preferably 4 N or more, more preferably 4.5 N or more, and still more preferably 4.7 N or more. The upper limit of the hardness of the plant-based cheese of the present invention is also not particularly limited, and is, for example, 8.5 N or less, 8 N or less, 7.5 or less, or 7 or less. Since the plant-based cheese of the present invention is excellent in hardness in a non-heated state, a molding process, such as a shred shape, a dice shape, or a slice shape, performed on a general animal milk cheese may be performed, or it may be a block shape that can be appropriately cut at the time of use. That is, the form of the plant-based cheese of the present invention may be any form found in a hard type cheese and a semi-hard type cheese in a general animal milk cheese.

Since the hardness of the plant-based cheese of the present invention is excellent in melting property, it can be used as a plant-based cheese for heat-cooking of heat-melting the plant-based cheese.

The type, used amount, and the like of the component to be contained in the plant-based cheese of the present invention are as described in the section of "1. Method for Producing Plant-Based Cheese".

Examples

Hereinafter, the present invention will be specifically described by means of Examples; however, the present invention is not to be construed as being limited to the following Examples.

[1. Enzyme Used]

Enzymes shown in the following table were used. All the enzymes used are manufactured by Amano Enzyme Inc.

[Table 1]

| Type | Origin | Manufacturer |
|---|---|---|
| Protease (filamentous fungus-derived protease) | *Aspergillus oryzae* | Amano Enzyme Inc. |
| Protease (bacteria-derived protease) | *Bacillus stearothermophilus* (currently reclassified into *Geobacillus stearothermophilus*) | Amano Enzyme Inc. |
| Glycosyltransferase (cyclodextrin-producing enzyme) | *Anoxybacillus caldiproteolyticus* | Amano Enzyme Inc. |
| Maltotriohydrolase | *Microbacterium imperiale* | Amano Enzyme Inc. |
| β-Amylase | *Bacillus flexus* | Amano Enzyme Inc. |
| α-Amylase | *Bacillus amyloliquefaciens* | Amano Enzyme Inc. |

[1-1. Protease Activity Measurement Method]

After 5 mL of 0.6% (v/w) casein solution (0.05 mol/L sodium hydrogen phosphate, pH 8.0 [in the case of a bacterial protease] or 0.7% (v/w) lactic acid, pH 3.0 [in the case of a filamentous fungus protease]) was warmed at 37°C for 10 minutes, 1 mL of a sample solution containing a protease was added, and the mixture was immediately shaken. After this solution was left to stand at 37°C for 10 minutes, 5 mL of a trichloroacetic acid solution (containing 1.8% trichloroacetic acid, 1.8% sodium acetate, and 0.33 mol/L acetic acid [in the case of a bacterial protease] or 0.44 mol/L of trichloroacetic acid [in the case of a filamentous fungus protease]) was added, the mixture was shaken, and left to stand at 37°C for 30 minutes again, and the mixture was filtered. 3 mL of the first filtrate was removed, 2 mL of the next filtrate was weighed, 5 mL of a 0.55 mol/L sodium carbonate solution and 1 mL of Folin's reagent (1 → 3) were added, and the mixture was shaken well and left to stand at 37°C for 30 minutes. An absorbance AT of this solution (enzymatic reaction solution) at a wavelength of 660 nm was measured using water as a control. Note that the Folin's reagent is a solution prepared by the following method. 20 g of sodium tungstate(VI) dihydrate and 5 g of disodium molybdate(VI) dihydrate are weighed, and put in a 300 mL flask, and about 140 mL of water, 10 mL of phosphoric acid (17 → 20), and 20 mL of hydrochloric acid are added, then a ground glass joint reflux condenser is attached, and the mixture is gently boiled for 10 hours. Next, 30 g of lithium sulfate monohydrate and 10 mL of water are added, and a very small amount of bromine is further added to make the dark green liquid yellow, and the mixture is boiled for 15 minutes without a condenser to remove an excessive amount of bromine. After cooling, water is added to make 200 mL, and the mixture is filtered with filter paper for qualitative analysis (2 kinds), and sealed and stored.

Separately, an absorbance AB of a solution (blank) obtained by performing the same operation as in the above-described enzymatic reaction solution was measured, except that 1 mL of a sample solution containing a protease was weighed, 5 mL of a trichloroacetic acid solution (containing 1.8% trichloroacetic acid, 1.8% sodium acetate, and 0.33 mol/L acetic acid [in the case of a bacterial protease] or 0.44 mol/L of trichloroacetic acid [in the case of a filamentous fungus protease]) was added and shaken, 5 mL of a 0.6% (v/w) casein solution (0.05 mol/L sodium hydrogen phosphate, pH 8.0 [in the case of a bacterial protease] or 0.7% (v/w) lactic acid, pH 3.0 [in the case of a filamentous fungus protease]) was then added and shaken, and the mixture was left to stand at 37°C for 30 minutes. The amount of enzyme that causes an increase in colored materials by Folin's reagent corresponding to 1 $\mu$g of tyrosine per minute was defined as 1 unit (1 U).

Each of 1 mL, 2 mL, 3 mL, and 4 mL of a 1 mg/mL tyrosine standard stock solution (0.2 mol/L of hydrochloric acid) was weighed, and a 0.2 mol/L hydrochloric acid solution was added thereto to make 100 mL. 2 mL of each solution was weighed, 5 mL of a 0.55 mol/L sodium carbonate solution and 1 mL of Folin's reagent (1 → 3) were added, and the mixture was immediately shaken and left to stand at 37°C for 30 minutes. For these solutions, absorbances A1, A2, A3, and A4 at a wavelength of 660 nm were measured using, as a control, a solution obtained by weighing 2 mL of a 0.2 mol/L hydrochloric acid solution and performing the same operation as described above. The absorbances A1, A2, A3, and A4 were plotted on the vertical axis and the amount ($\mu$g) of tyrosine in 2 mL of each solution was plotted on the horizontal axis to prepare a calibration curve, and the amount ($\mu$g) of tyrosine with respect to the absorbance difference of 1 was determined.

[Mathematical Formula 1]

$$\text{Protease activity (U/g, U/mL)} = (AT - AB) \times F \times 11/2 \times 1/10 \times 1/M$$

AT: Absorbance of enzymatic reaction solution
AB: Absorbance of blank
F: Tyrosine amount ($\mu$g) when absorbance difference determined from tyrosine calibration curve is 1
11/2: Conversion coefficient to total liquid amount after stop of reaction
1/10: Conversion coefficient per minute of reaction time
M: Amount (g or mL) of sample in 1 mL of sample solution

[1-2. Glycosyltransferase (Cyclodextrin-Producing Enzyme) Activity Measurement Method]

20 mL of water was added to 1.0 g of potato starch, and 5 mL of a sodium hydroxide reagent (1 mol/L) was gradually added while stirring to form a paste. The mixture was heated in a boiling water bath for 3 minutes while stirring, then 25 mL of water was added, and the mixture was cooled with running water, then pH was adjusted to 5.5 with an acetic acid reagent (1 mol/L), and water was further added to obtain 100 mL of a substrate solution. 10 mL of the substrate solution was weighed and warmed at 40°C for 10 minutes, and 1 mL of an enzyme solution was added and mixed. The mixture was incubated at 40°C for 10 minutes, and then 10 mL of a hydrochloric acid reagent (0.1 mol/L) was added to stop the reaction. To 1 mL of this reaction solution, 10 mL of an iodine-potassium iodide reagent (0.4 mmol/L) was added and mixed to obtain a test solution. Note that the iodine-potassium iodide reagent was prepared by dissolving 10.0 g of potassium iodide and 1.0 g of iodine in water to make 100 mL, and then diluting the solution 200 times with water. A solution prepared using water instead of the reaction solution was taken as a comparative solution, and the absorbance at 660 nm of each of the test solution and the comparative solution was measured. The amount of enzyme that reduces the blue iodine color of starch by 1 % per minute was defined as 1 unit (U), and the activity of the enzyme was calculated by the following formula.

[Mathematical Formula 2]

$$\text{Enzyme activity (U/g)} = (A0 - A10)/A0 \times 100/10 \times n$$

A10: Absorbance of reaction solution
A0: Absorbance of blank solution

100: % conversion coefficient
10: Reaction time (min)
n: Dilution factor per 1 g or 1 mL of sample

[1-3. Maltotriohydrolase Activity Measurement Method]

[Activity Measurement Method of Maltotriohydrolase]

25 g of sodium carbonate, 25 g of (+)-potassium sodium tartrate tetrahydrate, 20 g of sodium hydrogen carbonate, and 200 g of sodium sulfate were weighed and dissolved by adding water to make 1000 mL, thereby preparing a first solution. 30 g of copper(II) sulfate pentahydrate was weighed and added in 150 mL of water to be dissolved, four drops of sulfuric acid were added, and water was further added to make 200 mL, thereby preparing a second solution. At the time of use, a solution obtained by mixing 25 volumes of the first solution and 1 volume of the second solution was used as an alkaline copper reagent. The enzyme was allowed to act on the soluble starch as a substrate, and the resulting reducing sugar was subjected to colorimetric determination by the Somogyi-Nelson method. In a 50 mL Nessler tube, 0.5 mL of a soluble starch solution and 0.4 mL of a 0.1 mol/L acetic acid-sodium acetate buffer solution (pH 6.0) (containing 0.05 mol/L $CaCl_2$) were weighed, and shaken well, the mixture was left to stand at $40 \pm 0.5°C$ for 10 to 15 minutes, and then 0.1 mL of a sample solution was added thereto, and the mixture was immediately shaken. This solution was left to stand at $40 \pm 0.5°C$ for exactly 15 minutes, 1 mL of an alkaline copper reagent was then added, the mixture was shaken and capped, and the mixture was heated in a boiling water bath for exactly 20 minutes and immediately cooled. After cooling, 1 mL of a Nelson's solution was added, and the mixture was shaken well until a red precipitate of copper suboxide was completely dissolved, the mixture was then left to stand at room temperature for 20 minutes, 22 mL of water was added, and the mixture was shaken well. The absorbance of this solution at a wavelength of 520 nm was measured using water as a control. The amount of enzyme that produces a reducing sugar corresponding to 1 μmol of glucose per minute was defined as 1 unit.

[1-4. β-Amylase Activity Measurement Method]

(1) A potato starch was used as a substrate, the potato starch was previously dried at 105°C for 2 hours, 1.0 g of the dried product thereof was weighed, 20 mL of water was added, and 5 mL of a sodium hydroxide reagent (2 mol/L) was gradually added while stirring to form a paste. Next, the paste was heated in a water bath for 3 minutes with stirring, and then 25 mL of water was added. After cooling, the mixture was neutralized by adding a hydrochloric acid reagent (2 mol/L) and a hydrochloric acid reagent (0.1 mol/L), 10 mL of a 1 mol/L acetic acid-sodium acetate buffer solution (pH 5.0) was added, and water was further added to make 100 mL, thereby obtaining a substrate solution.
(2) 10 mL of the substrate solution was weighed and warmed at 37°C for 10 minutes, 1 mL of a sample solution was added and immediately shaken, the mixture was warmed at the same temperature for 10 minutes or 30 minutes, 4 mL of a Fehling's reagent was added and shaken lightly, the mixture was heated in a water bath for 15 minutes, and then cooled to 25°C or lower, and 2 mL of a potassium iodide reagent (for a β-amylase-invertase activity test) and 2 mL of sulfuric acid (1 → 6) were added, thereby obtaining a test solution. Note that the Fehling's reagent is a solution prepared at the time of use by mixing a copper solution prepared by weighing 34.66 g of fine crystals of copper(II) sulfate pentahydrate and adding water thereto to make 500 mL and an alkaline tartrate solution prepared by weighing 173 g of (+)-potassium sodium tartrate tetrahydrate and 50 g of sodium hydroxide and adding water thereto to make 500 mL, at a ratio of 1 volume of the copper solution and 1 volume of the alkaline tartrate solution. The potassium iodide reagent (for a β-amylase-invertase activity test) is a 30 wt% potassium iodide solution. Sulfuric acid (1 → 6) is a 16.7 wt% sulfuric acid solution. Separately, the same operation as in the preparation of the test solution was performed using 10 mL of water instead of the substrate solution to prepare a comparative solution. For the test solution and the comparative solution, liberated iodine was titrated with a 0.05 mol/L sodium thiosulfate solution. The end point was set to a time at which 1 to 2 drops of a soluble starch reagent were added when the titration was close to the end point, and the resulting blue color disappeared.
(3) The amount of enzyme that leads to an increase in reducing power corresponding to 1 mg of glucose per minute was defined as 1 unit (1 U), and the activity of the β-amylase was calculated by the following formula.

[Mathematical Formula 3]

$$\beta\text{-Amylase activity (U/g, U/mL)} = \text{amount (mg) of glucose} \times 1/10 \times 1/M$$

Amount (mg) of glucose = (b - a) × 1.6 × f
a: Titration value (mL) of enzymatic reaction solution
b: Titration value (mL) of blank solution
1.6: 1 mL of 0.05 mol/L sodium thiosulfate solution corresponds to a glucose amount of 1.6 mg.
1/10: Unit conversion coefficient of reaction time (min)
M: Amount (g or mL) of sample in 1 mL of sample solution
f: Factor of 0.05 mol/L sodium thiosulfate solution (for quantification)

[1-5. α-Amylase Activity Measurement Method]

After 10 mL of a 1% potato starch substrate solution (0.1 mol/L of acetic acid (pH 5.0)) was warmed at 37°C for 10 minutes, 1 mL of a sample solution containing an α-amylase was then added, and the mixture was immediately shaken. This solution was left to stand at 37°C for 10 minutes, 1 mL of this solution was then added to 10 mL of a 0.1 mol/L hydrochloric acid reagent, and the mixture was immediately shaken. Next, 0.5 mL of this solution was weighed, 10 mL of 0.0002 mol/L iodine reagent (the Japanese pharmacopoeia) was added, the mixture was shaken, and then an absorbance (AT) at a wavelength of 660 nm was measured using water as a control. Separately, an absorbance (AB) was measured by the same operation adding 1 mL of water instead of the sample solution. The amount of enzyme that reduces the color of potato starch with iodine by 10% per minute was defined as 1 unit (1 U).

[2. Materials Used]

Commercially available materials shown in the following table were used.

[Table 2]

|  | Product name | Manufacturer |
|---|---|---|
| Pea protein material | Pea protein 870 MV (starch content: 4 wt%, protein content: 80 wt%) | PURIS |
| Tapioca starch | Tapioca Starch | EARTHBORN ELEMENT |
| Coconut oil | Refined COCONUT OIL | Nutiva |
| Canola oil | Expeller Pressed Non-GMO Canola Oil | NATIVE HARVEST |
| Tricalcium phosphate | Tricalcium phosphate | Eastchem |
| Lactic acid | Lactic acid 88% | Home Brew Ohio |
| κ-carrageenan | Kappa Carrageenan | CAPE CRYSTAL BRANDS |
| Salt | Ionized salt | MORTON |

[3. Production of Plant-Based Cheese]

[3-1. Examples 1 to 4 and Comparative Examples 1 to 4]

250 ml (50 parts by weight) of pure water (RO water) was put into a Thermomix mixer, and a group component (1) and a group enzyme (2) in Table 3 were added in the indicated amounts while stirring at 50°C and speed 3. The mixture was incubated at 50°C for 30 minutes, and then heated to 90°C to deactivate the enzyme. Thereafter, a group component (3) in Table 3 was added, and the mixture was incubated at 90°C for 15 minutes. The stirring was stopped, and each aluminum container (bottom inner diameter: diameter 5 cm) was filled with 100 g of the mixture, covered with a wrap film, and stored in a refrigerator (4°C) for 7 days. After the storage, the sample of the plant-based cheese was taken out from the refrigerator (4°C) and immediately cut into a 20-mm cube (dicing process). Thus, a diced plant-based cheese was obtained.

[3-2. Examples 5 and 6]

[Example 5]

250 ml (50 parts by weight) of pure water (RO water) was put into a Thermomix mixer, and a1, c, b, d, a21, and a22 in Table 5 were mixed in the indicated amounts while stirring at 50°C and speed 3, and incubated at 50°C for 30 minutes. Thereafter, the mixture was heated to 90°C to deactivate the enzymes (a21 and a22) in 10 minutes. Thereafter, each aluminum container (bottom inner diameter: diameter 5 cm) was filled with 100 g of the mixture, covered with a wrap film, and stored in a refrigerator (4°C) for 7 days. After the storage, the sample of the plant-based cheese was taken out from the refrigerator (4°C) and immediately cut into a 20-mm cube (dicing process). Thus, a diced plant-based cheese was obtained.

[Example 6]

250 ml (50 parts by weight) of pure water (RO water) was put into a Thermomix mixer, a1 and a21 in Table 5 were mixed in the indicated amounts while stirring at 50°C and speed 3, and incubated at 50°C for 30 minutes, and the mixture was heated to 90°C to deactivate the enzyme (a21) in 10 minutes. Thereafter, the temperature was lowered from the deactivation temperature (90°C) to the reaction temperature (50°C), c and a22 in Table 5 were added, and the mixture was incubated at 50°C for 30 minutes. Thereafter, the mixture was heated to 90°C to deactivate the enzyme (a22) in 10 minutes. Thereafter, b and d in Table 5 were added, and the mixture was incubated at 90°C for 10 minutes. The stirring was stopped, and each aluminum container (bottom inner diameter: diameter 5 cm) was filled with 100 g of the mixture, covered with a wrap film, and stored in a refrigerator (4°C) for 7 days. After the storage, the sample of the plant-based cheese was taken out from the refrigerator (4°C) and immediately cut into a 20-mm cube (dicing process). Thus, a diced plant-based cheese was obtained.

[4. Evaluation of Plant-Based Cheese]

[4-1. Melting Property]

A dice piece of the diced plant-based cheese was heated for 30 seconds using a microwave oven, and the state after heating was evaluated in four stages according to the following criteria. The results are shown in Tables 3 and 5.

--: Melting of the cheese piece cannot be confirmed.
-: The cheese piece is slightly melted and the shape thereof clearly remains.
+: Although the shape of the cheese piece slightly remains, melting can be clearly confirmed.
++: The cheese piece is melted to such an extent that little shape thereof remains.
+++: A melt of the cheese piece easily flows.

[4-2. Texture Analysis]

The hardness and cohesiveness of the dice piece of the diced plant-based cheese were analyzed. Specifically, using a cylindrical probe (diameter: 12.7 mm), two compression-decompression cycles were performed at a speed of 1 mm/s with a time interval of 5 seconds. The probe compressed the sample from an initial height (25 mm) by 7.5 mm (Deformation: 30%). Analysis was performed in triplicate. Texture profile analysis (TPA) was performed according to Texture Analyzer (Multi Test 2.5-i manufactured by Mecmesin) (n = 3). The results are shown in Table 3. Relative values of the hardness of the plant-based cheeses of Examples 5 and 6 were derived when the hardness of the plant-based cheese of Example 1 was taken as 1. The results are shown in Table 5.

[4-3. Sensory Evaluation]

A sensory test was performed by four trained panelists in a blind test. In the sensory test, the effect of reducing the flavor of the pea as a raw material was rated based on the following criteria, and the rating was averaged to derive the raw material flavor reduction score. The results are shown in Table 4.

1: The effect of reducing the bean flavor is not felt at all.

2: The effect of reducing the bean flavor is not felt so much.

3: The effect of reducing the bean flavor is slightly felt.

4: The effect of reducing the bean flavor is clearly felt.

[5. Result]

[Table 3]

| | | Comparative Example 1 | Comparative Example 2 | Comparative Example 3 | Example 1 | Example 2 | Example 3 | Example 4 | Comparative Example 4 |
|---|---|---|---|---|---|---|---|---|---|
| (1) | RO water | 50 | 50 | 50 | 50 | 50 | 50 | 50 | 50 |
| | Pea protein material | 18.8 | 18.8 | 18.8 | 18.8 | 18.8 | 18.8 | 18.8 | 18.8 |
| | <Protein in Material> | <15> | <15> | <15> | <15> | <15> | <15> | <15> | <15> |
| | <Starch in Material> | <0.75> | <0.75> | <0.75> | <0.75> | <0.75> | <0.75> | <0.75> | <0.75> |
| | Lactic acid | 0.4 | 0.4 | 0.4 | 0.4 | 0.4 | 0.4 | 0.4 | 0.4 |
| (2) | Filamentous fungus-derived protease | - | [935 U/g] | - | [935 U/g] | [935 U/g] | [935 U/g] | [935 U/g] | [935 U/g] |
| | Bacteria-derived protease | - | [280 U/g] | - | [280 U/g] | [280 U/g] | [280 U/g] | [280 U/g] | [280 U/g] |
| | Cyclodextrin-producing enzyme | - | - | {40 U/g} | {40 U/g} | {80 U/g} | - | - | - |
| | Maltotriohydrolase | - | - | - | - | - | {1.2 U/g} | - | - |
| | β-Amylase | - | - | - | - | - | - | {0.65 U/g} | - |
| | α-Amylase | - | - | - | - | - | - | - | {1300 U/g} |
| (3) | Tapioca starch | 10 | 10 | 10 | 10 | 10 | 10 | 10 | 10 |
| | Canola oil | 2 | 2 | 2 | 2 | 2 | 2 | 2 | 2 |
| | Coconut oil | 16 | 16 | 16 | 16 | 16 | 16 | 16 | 16 |
| | Tricalcium phosphate | 1.4 | 1.4 | 1.4 | 1.4 | 1.4 | 1.4 | 1.4 | 1.4 |
| | Common salt | 0.6 | 0.6 | 0.6 | 0.6 | 0.6 | 0.6 | 0.6 | 0.6 |
| | κ-carrageenan | 0.8 | 0.8 | 0.8 | 0.8 | 0.8 | 0.8 | 0.8 | 0.8 |
| | Melting property | -- | ++ | - | ++ | ++ | + | ++ | ++ |
| | Hardness (N) | 20.79 | 2.48 | 9 | 4.85 | 4.79 | 6.85 | 3.38 | 2.88 |
| | Cohesiveness | 0.29 | 0.32 | 0.28 | 0.37 | 0.37 | 0.36 | 0.27 | 0.21 |

In the table, the numerical value indicating the blending amount of each component is parts by weight.

The numerical value represented by [] indicating the blending amount of the enzyme is an activity value per 1 g of the protein contained in the protein material.

The numerical value represented by {} indicating the blending amount of the enzyme is an activity value per 1 g of the starch contained in the protein material.

[Table 4]

|  | Raw material flavor reduction score |
| --- | --- |
| Comparative Example 1 | 1.25 |
| Example 1 | 2.75 |
| Example 2 | 3.5 |

As is apparent from Table 3, the melting property was remarkably improved in the plant-based cheese produced only by the hydrolysis treatment (protease treatment) (Comparative Example 2) as compared with the plant-based cheese produced without the hydrolysis treatment and the predetermined enzymatic treatment (Comparative Example 1), but the hardness was remarkably decreased, and in the plant-based cheese produced only by the predetermined enzymatic treatment (treatment with the cyclodextrin-producing enzyme) (Comparative Example 3), although excellent hardness was obtained as compared with Comparative Example 2, satisfactory melting property was not obtained, and both the melting property and the hardness were not achieved. In the plant-based cheese (Comparative Example 4) produced by performing the hydrolysis treatment (protease treatment) and the treatment with an $\alpha$-amylase, satisfactory hardness was not obtained, and the melting property and the hardness were not achieved.

On the other hand, in the plant-based cheese produced by performing the hydrolysis treatment (protease treatment) and the predetermined enzymatic treatment (treatment with a cyclodextrin-producing enzyme, a maltotriohydrolase, or a $\beta$-amylase) (Examples 1 to 4), both the melting property and the hardness were achieved. Among them, in particular, in the plant-based cheese produced by performing the treatment with a cyclodextrin-producing enzyme or a maltotriohydrolase as the predetermined enzymatic treatment (Examples 1 to 3), excellent cohesiveness was also obtained, and processing characteristics were further improved.

As is apparent from Table 4, the effect of reducing the flavor of peas as a raw material was observed in the plant-based cheese produced by performing the hydrolysis treatment (protease treatment) and the predetermined enzymatic treatment (cyclodextrin-producing enzyme) (Examples 1 and 2), as compared with the plant-based cheese produced without performing the hydrolysis treatment and the predetermined enzymatic treatment (Comparative Example 1).

[Table 5]

|  |  |  | Example 1 | Example 5 | Example 6 |
| --- | --- | --- | --- | --- | --- |
| RO water |  |  | 50 | 50 | 50 |
| a1 | Pea protein material |  | 18.8 | 18.8 | 18.8 |
|  |  | <Protein in Material> | <15> | <15> | <15> |
|  |  | <Starch <Starch in Material> | <0.75> | <0.75> | <0.75> |
| C | Lactic acid |  | 0.4 | 0.4 | 0.4 |
| a21 | Filamentous fungus-derived protease |  | [935 U/g] | [935 U/g] | [935 U/g] |
| a21 | Bacteria-derived protease |  | [280 U/g] | [280 U/g] | [280 U/g] |
| a22 | Cyclodextrin-producing enzyme |  | {40 U/g} | {40 U/g} | {40 U/g} |
| b | Tapioca starch |  | 10 | 10 | 10 |
| d | Canola oil |  | 2 | 2 | 2 |
| d | Coconut oil |  | 16 | 16 | 16 |

(continued)

| | | Example 1 | Example 5 | Example 6 |
|---|---|---|---|---|
| d | Tricalcium phosphate | 1.4 | 1.4 | 1.4 |
| d | Common salt | 0.6 | 0.6 | 0.6 |
| d | κ-carrageenan | 0.8 | 0.8 | 0.8 |
| Procedure overview | | Step (A) { (a1+c +a21+a22) ↓ Step (B) { +(b+d) | Steps (A), (B) { (a1+c +b+d +a21 +a22) | Step (A) { (a1+a21) ↓ +(c+a22) ↓ Step (B) { +(b+d) |
| Melting property | | ++ | +++ | +++ |
| Hardness (N) reference value | | 1 | 1.23 | 1.28 |

In the table, the numerical value indicating the blending amount of each component is parts by weight.

The numerical value represented by [] indicating the blending amount of the enzyme is an activity value per 1 g of the protein contained in the protein material.

The numerical value represented by {} indicating the blending amount of the enzyme is an activity value per 1 g of the starch contained in the protein material.

As shown in the comparison between Example 1 and Example 5 in Table 5, when the hydrolysis treatment (treatment with a21) and the predetermined enzymatic treatment (treatment with a22) were simultaneously performed in the step (A), the hardness in a non-heated state was improved and the melting property was also excellent in the case of simultaneously performing the step (A) and the step (B) (Example 5), and thus the compatibility between the hardness in a non-heated state and the melting property was further excellent.

As shown in the comparison between Example 1 and Example 6 in Table 5, when the step (A) and the step (B) were performed in this order, the hardness in a non-heated state was improved and the melting property was also excellent in the case of performing the hydrolysis treatment (treatment with a21) and the predetermined enzymatic treatment (treatment with a22) in this order in the step (A) (Example 6), and thus the compatibility between the hardness in a non-heated state and the melting property was further excellent.

**Claims**

1.    A method for producing a plant-based cheese, the production method comprising:

(A) a step for subjecting a material containing a plant protein to a hydrolysis treatment and an enzymatic treatment with an enzyme selected from the group consisting of a glycosyltransferase, a maltotriohydrolase, and a β-amylase; and
(B) a step for mixing starch with the material.

2.    The production method according to claim 1, wherein the glycosyltransferase is a cyclodextrin-producing enzyme.

3.    The production method according to claim 1, wherein the hydrolysis treatment is an enzymatic treatment with a protease.

4.    The production method according to claim 3, wherein the protease is a filamentous fungus-derived protease and/or a bacteria-derived protease.

5.    The production method according to claim 1, comprising a step for deactivating the enzyme used in the step (A) after the step (A) and before the step (B).

6.    The production method according to claim 3, wherein a used amount of the protease per 1 g of the plant protein in the step (A) is 5 to 300000 U.

7. The production method according to claim 1, wherein a used amount of the glycosyltransferase per 1 g of the plant protein in the step (A) is 0.5 to 15000 U.

8. The production method according to claim 1, wherein a used amount of the maltotriohydrolase per 1 g of the plant protein in the step (A) is 0.01 to 30 U.

9. The production method according to claim 1, wherein a used amount of the $\beta$-amylase per 1 g of the plant protein in the step (A) is 0.005 to 15 U.

10. The production method according to claim 1, wherein a used amount of the starch in the step (B) is 0.2 to 1 part by weight per 1 part by weight of the plant protein.

11. A method for imparting hardness and melting property of a plant-based cheese, the imparting method comprising, in production of a plant-based cheese:

   (A) a step for subjecting a material containing a plant protein to a hydrolysis treatment and an enzymatic treatment with an enzyme selected from the group consisting of a glycosyltransferase, a maltotriohydrolase, and a $\beta$-amylase; and
   (B) a step for mixing starch with the material.

12. A plant-based cheese obtained by the production method according to claim 1.

## INTERNATIONAL SEARCH REPORT

| | International application No. |
|---|---|
| | **PCT/JP2024/016326** |

| A. | CLASSIFICATION OF SUBJECT MATTER |
|---|---|

*A23C 20/02*(2021.01)i; *C12N 9/10*(2006.01)i; *C12N 9/26*(2006.01)i; *C12N 9/52*(2006.01)i; *C12N 9/58*(2006.01)i
FI:   A23C20/02; C12N9/26 A; C12N9/10; C12N9/52; C12N9/58

According to International Patent Classification (IPC) or to both national classification and IPC

| B. | FIELDS SEARCHED |
|---|---|

Minimum documentation searched (classification system followed by classification symbols)

A23C20/00-20/02; C12N9/00-9/99; A23L5/40-5/49; A23L31/00-33/29

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | WO 2022/181810 A1 (AMANO ENZYME U.S.A. CO., LTD., AMANO ENZYME INC.) 01 September 2022 (2022-09-01)<br>claim 1, paragraphs [0007], [0063] | 1-12 |
| Y | WO 2012/111327 A1 (GLICO NUTRITION CO., LTD.) 23 August 2012 (2012-08-23)<br>paragraphs [0004], [0073], [0132]-[0133], [0179], [0250] | 1-12 |
| Y | JP 2020-015871 A (SHOWA SANGYO CO., LTD.) 30 January 2020 (2020-01-30)<br>paragraphs [0017], [0029], example 1 | 1-12 |
| Y | WO 2011/007404 A1 (AMANO ENZYME INC.) 20 January 2011 (2011-01-20)<br>claims 1-3, paragraphs [0069], [0072], [0075] | 1-12 |
| A | WO 2006/135089 A1 (FUJI OIL CO., LTD.) 21 December 2006 (2006-12-21)<br>entire text | 1-12 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **09 July 2024** | **23 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/016326**

| C. | DOCUMENTS CONSIDERED TO BE RELEVANT | |
|---|---|---|
| Category* | Citation of document, with indication, where appropriate, of the relevant passages | Relevant to claim No. |
| A | WO 2021/193892 A1 (FUJI OIL HOLDINGS INC., FUJI OIL CO., LTD.) 30 September 2021 (2021-09-30)<br>    entire text | 1-12 |

## INTERNATIONAL SEARCH REPORT
### Information on patent family members

| International application No. |
| :--- |
| **PCT/JP2024/016326** |

| Patent document cited in search report | | | Publication date (day/month/year) | Patent family member(s) | Publication date (day/month/year) |
| :--- | :--- | :--- | :--- | :--- | :--- |
| WO | 2022/181810 | A1 | 01 September 2022 | EP 4298914 A1 claim 1, paragraphs [0007], [0073] CN 116940240 A | |
| WO | 2012/111327 | A1 | 23 August 2012 | (Family: none) | |
| JP | 2020-015871 | A | 30 January 2020 | (Family: none) | |
| WO | 2011/007404 | A1 | 20 January 2011 | US 2012/0121760 A1 claims 1-3, paragraphs [0082], [0085], [0088] JP 2011-36237 A EP 2454942 A1 CN 102595910 A KR 10-2012-0052263 A | |
| WO | 2006/135089 | A1 | 21 December 2006 | US 2008/0213428 A1 entire text | |
| WO | 2021/193892 | A1 | 30 September 2021 | US 2023/0094989 A1 entire text JP 2023-115335 A EP 4129076 A1 CN 115334890 A KR 10-2022-0160574 A | |

Form PCT/ISA/210 (patent family annex) (July 2022)

**REFERENCES CITED IN THE DESCRIPTION**

**Patent documents cited in the description**

- WO 2022181810 A